# DEMANDE DE BREVET EUROPEEN

(11) **EP 3 839 012 A1**
(43) Date de publication de la demande: **23.06.2021**
(21) Numéro de dépôt: 20210040.0
(22) Date de dépôt: 26.11.2020
(51) Int. Cl.: C10G 11/18, C10G 9/36, C10G 51/04, C10G 45/32, C10G 21/00

(54) **DISPOSITIF ET PROCÉDÉ DE PRODUCTION D OLÉFINES LÉGÈRES PAR CRAQUAGE CATALYTIQUE ET VAPOCRAQUAGE**

(30) Priorité: 16.12.2019 FR 1914509
(71) Demandeur: IFP Energies nouvelles, 92852 Rueil-Malmaison (FR)
(72) Inventeur: THINON, Olivier, 92852 RUEIL-MALMAISON CEDEX (FR); PIERRON, Anne-Claire, 92852 RUEIL-MALMAISON CEDEX (FR); SANTOS-MOREAU, Vania, 92852 RUEIL-MALMAISON CEDEX (FR)
(74) Mandataire: IFP Energies nouvelles

(57) **Abrégé**

La présente invention concerne un dispositif et un procédé NCC de production d'oléfines légères et d'aromatiques, dans lesquels la coupe comprenant de l'éthane et/ou du propane (12) de l'effluent de craquage est envoyée au moins en partie dans un four de vapocraquage (19) alimenté par de la vapeur d'eau (20) pour produire un effluent de vapocraquage (21) comprenant de l'éthylène et/ou du propylène.

## Description

### Domaine technique

La présente invention concerne un dispositif et un procédé couramment appelés NCC (abréviation de Craquage Catalytique de Naphta ou « Naphtha Catalytic Cracking » selon la terminologie anglo-saxonne) de production d'oléfines légères (C2-C4), et plus particulièrement d'éthylène, propylène et optionnellement d'aromatiques, à partir d'une charge d'hydrocarbures, principalement des naphtas et essences par conversion catalytique. Le besoin de carburants à faibles émissions a créé une demande accrue d'oléfines légères à utiliser dans les procédés d'alkylation, d'oligomérisation, de synthèse de méthyl tert-butyl éther (MTBE) et d'éthyl tert-butyl éther (ETBE). En outre, la demande en oléfines légères, en particulier en éthylène et en propylène, à faible coût, continue d'être recherchée pour servir de matière première pour la production de polyoléfines, en particulier de polyéthylène et de polypropylène.

### Technique antérieure

Le vapocraquage (« Steam Cracking » selon la terminologie anglo-saxonne), la déshydrogénation du propane, la métathèse, le MTO (« Méthanol to Olefins » selon la terminologie anglo-saxonne) ou la technologie FCC (« Fluid Catalytic Cracking » selon la terminologie anglo-saxonne) sont des procédés qui produisent pratiquement la totalité de l'éthylène et du propylène nécessaires pour répondre à la demande mondiale. Les hydrocarbures utilisés comme matière première pour la production d'oléfines légères comprennent le butane, le propane, les condensats du gaz naturel, les coupes hydrocarbonées liquides issues de la distillation du pétrole et les matières carbonées y compris le charbon, les matières plastiques recyclées ou toute matière organique.

Ainsi, l'intérêt relativement récent de la mise en œuvre d'un dispositif et d'un procédé de production d'oléfines légères par craquage catalytique de charge naphta provient de la nécessité de disposer d'oléfines légères pour la pétrochimie, en plus de la source traditionnelle que constitue le vapocraquage de naphta, du fait de :
- la présence d'un déséquilibre qui va croissant entre la production de propylène et la production d'éthylène, surtout avec le succès des vapocraqueurs sur charge éthane moins producteur de propylène,
- la disponibilité pressentie de naphta du fait de la réduction de la consommation d'essence, de l'introduction de carburants alternatifs, et de l'arrivée massive sur le marché de condensats (e.g. issus de shale), et
- la volonté de réduction des coûts énergétiques de production des oléfines et en particulière du propylène et la réduction des émissions de CO₂ qui découlent d'une opération à plus basse température que celle d'un vapocraquage de naphta, l'activité catalytique réduisant le besoin de fortes températures.

Le procédé NCC a été développé pour la production d'oléfines légères. Il est généralement opéré en présence d'un catalyseur dans la plupart des cas zéolitique à une température de l'ordre de 600°C pour maximiser le rendement en oléfines légères et en particulier en propylène. Selon les configurations, le procédé NCC permet de potentiellement augmenter la quantité de propylène produit par tonne de naphta et en certains cas de produire plus de propylène que le vapocraqueur ; de mieux valoriser le naphta en produits à forte valeur ajoutée dont la demande est sans cesse croissante, et en particulier en propylène, éthylène et BTX ; et de craquer le naphta à une température 150 à 200°C plus basse que celle utilisée pour le vapocraquage thermique opérant généralement entre 800 et 900°C, de manière à avoir un coût énergétique de production de propylène et d'éthylène plus faible tout en réduisant les émissions de CO₂.

Le vapocraqueur est un des principaux procédés utilisés actuellement pour produire des oléfines légères à partir de naphta et de gaz naturel. Ce procédé, étant un procédé thermique opéré à hautes températures, produit davantage d'éthylène et selon les conditions d'opération le ratio massique éthylène/propylène est souvent de l'ordre de 2. Quand les charges à traiter sont des charges liquides présentant des teneurs importantes en oléfines des difficultés opérationnelles peuvent avoir lieu avec la formation de quantités importantes de coke. Egalement, ce procédé nécessite des taux de dilution avec de la vapeur conséquents.

Avec l'intérêt croissant pour la production d'oléfines légères des unités FCC ont été opérées de manière à maximiser la production de ces composés (voir par exemple US4830728, US6489530A, US2006108261A, CN102746888A). La difficulté de produire des oléfines légères par des procédés FCC est le fait qu'il est très difficile de maximiser de rendement en oléfines légères tout en maintenant une conversion importante.

D'autres procédés qui permettent de transformer des charges liquides du type naphta ou essence en oléfines légères et en particulier en propylène ont été développés en ayant comme objectif d'augmenter la conversion et le rendement en oléfines légères (voir par exemple US2009288985A, US2009143629A, et US3776838A).

### Résumé de l'invention

Un premier objet de la présente description est de fournir un procédé permettant d'améliorer la production des oléfines légères (C2-C4) et optionnellement des BTX à partir de Naphta.

La présente invention permet de maximiser le rendement en oléfines légères en envoyant la coupe éthane et/ou propane dans un vapocraqueur.

Selon un premier aspect, la présente invention concerne un procédé de production d'oléfines ayant un nombre d'atomes de carbone compris entre 2 et 4 par craquage catalytique, comprenant les étapes suivantes :
- mettre en contact toute ou partie d'une charge comprenant du naphta, un catalyseur et un diluant dans un réacteur de craquage catalytique de naphta pour convertir au moins en partie la charge comprenant du naphta en oléfines ayant un nombre d'atomes de carbone compris entre 2 et 4 et produire un effluent de craquage ;
- envoyer l'effluent de craquage dans une première section de séparation pour produire au moins les fractions suivantes :
   ∘ coupe comprenant de l'éthane et/ou du propane,
   ∘ coupe comprenant de l'éthylène, et
   ∘ coupe comprenant du propylène ; et
- envoyer au moins en partie la coupe comprenant de l'éthane et/ou du propane dans un four de vapocraquage alimenté par de la vapeur d'eau pour produire un effluent de vapocraquage comprenant de l'éthylène et/ou du propylène.

Selon un ou plusieurs modes de réalisation, la coupe comprenant de l'éthane et/ou du propane est additionnée à la vapeur d'eau selon un ratio massique [vapeur d'eau]/[coupe comprenant de l'éthane et/ou du propane] entre 0,2 et 0,8, et subit un craquage à une température comprise entre 750 et 900°C dans le four de vapocraquage.

Selon un ou plusieurs modes de réalisation, l'effluent de vapocraquage est envoyé dans la première section de séparation.

Selon un ou plusieurs modes de réalisation, la première section de séparation produit au moins une coupe comprenant des composés comprenant 4 atomes de carbone, et au moins une partie de la coupe comprenant des composés comprenant 4 atomes de carbone est recyclée vers le four de vapocraquage.

Selon un ou plusieurs modes de réalisation, la première section de séparation produit au moins une coupe comprenant des composés comprenant 4 atomes de carbone, et au moins une partie de la coupe comprenant des composés comprenant 4 atomes de carbone est recyclée vers le réacteur de craquage catalytique de naphta.

Selon un ou plusieurs modes de réalisation, la première section de séparation produit en outre au moins une des fractions suivantes :
∘ coupe comprenant de l'hydrogène,
∘ coupe comprenant du méthane,
∘ coupe comprenant des composés comprenant 4 atomes de carbone, et
∘ coupe comprenant des composés comprenant au moins 5 atomes de carbone comprenant des paraffines, des oléfines et des aromatiques.

Selon un ou plusieurs modes de réalisation, le réacteur de craquage catalytique de naphta est opéré en lit fluidisé turbulent.

Selon un ou plusieurs modes de réalisation, le catalyseur comprend au moins une zéolithe, et le réacteur de craquage catalytique de naphta est utilisé selon les conditions opératoires suivantes : une température comprise entre 500 et 700°C ; une pression totale absolue comprise entre 0,1 et 0,5 MPa ; un temps de contact entre la charge comprenant du naphta et le catalyseur compris entre 200 millisecondes et 20 secondes ; et une pression partielle des hydrocarbures dans la charge comprenant du naphta comprise entre 0,02 et 0,3 MPa.

Selon un ou plusieurs modes de réalisation, un catalyseur coké est extrait du réacteur de craquage catalytique de naphta, est séparé de l'effluent de craquage, est strippé et est régénéré dans un régénérateur pour produire un catalyseur régénéré recyclé dans le réacteur de craquage catalytique de naphta.

Selon un ou plusieurs modes de réalisation, la première section de séparation produit au moins une coupe comprenant du méthane, et la coupe comprenant du méthane est au moins en partie utilisée comme gaz combustible alimentant le régénérateur.

Selon un ou plusieurs modes de réalisation, la première section de séparation produit en outre au moins une coupe comprenant de l'éthane et/ou du propane, et la coupe comprenant de l'éthane et/ou du propane est au moins en partie utilisée comme gaz combustible alimentant le régénérateur.

Selon un ou plusieurs modes de réalisation, la première section de séparation produit au moins une coupe comprenant des composés comprenant au moins 5 atomes de carbone comprenant des paraffines, des oléfines et des aromatiques, le procédé comprenant en outre les étapes suivantes :
- envoyer au moins une partie de la coupe comprenant des composés comprenant au moins 5 atomes de carbone dans une deuxième section de séparation pour produire les fractions suivantes :
   ∘ coupe comprenant des composés comprenant 5 atomes de carbone, et
   ∘ coupe comprenant des composés comprenant au moins 6 atomes de carbone riche en aromatiques ;
- envoyer au moins une partie de la coupe comprenant des composés comprenant au moins 6 atomes de carbone dans une unité d'extraction des aromatiques pour produire les fractions suivantes :
   ∘ coupe enrichie en aromatiques, et
   ∘ coupe pauvre en aromatiques ; et
- recycler au moins en partie la coupe comprenant des composés comprenant 5 atomes de carbone dans le réacteur de craquage catalytique de naphta.

Selon un ou plusieurs modes de réalisation, le procédé comprend en outre l'étape suivante :
- envoyer au moins une partie de la coupe enrichie en aromatiques (31) dans une unité de séparation des aromatiques (33) pour produire au moins les fractions suivantes :
   ∘ fraction riche en benzène (34),
   ∘ fraction riche en toluène (35), et
   ∘ au moins une fraction riche en xylènes (36).

Selon un ou plusieurs modes de réalisation, la coupe pauvre en aromatiques est recyclée au moins en partie dans le réacteur de craquage catalytique de naphta.

Selon un ou plusieurs modes de réalisation, la charge comprenant du naphta comprend des aromatiques et toute ou partie de la charge comprenant du naphta est envoyée dans la section de séparation ou dans l'unité d'extraction des aromatiques.

Selon un ou plusieurs modes de réalisation, l'unité d'extraction des aromatiques comprend une section d'hydrogénation des oléfines pour produire un effluent d'hydrogénation pauvre en oléfines et comprenant des paraffines et des aromatiques ; et une section d'extraction pour séparer l'effluent d'hydrogénation et produire la coupe enrichie en aromatiques, et la coupe pauvre en aromatiques.

Selon un deuxième aspect, la présente invention concerne un dispositif de production d'oléfines ayant un nombre d'atomes de carbone compris entre 2 et 4 et d'aromatiques par craquage catalytique, comprenant les unités suivantes :
- un réacteur de craquage catalytique de naphta adapté pour mettre en contact toute ou partie d'une charge comprenant du naphta, un catalyseur et un diluant, convertir au moins en partie la charge comprenant du naphta en oléfines ayant un nombre d'atomes de carbone compris entre 2 et 4 et produire un effluent de craquage ;
- une première section de séparation adaptée pour traiter l'effluent de craquage et produire au moins les fractions suivantes :
   ∘ coupe comprenant de l'éthane et/ou du propane,
   ∘ coupe comprenant de l'éthylène, et
   ∘ coupe comprenant du propylène ;
- un four de vapocraquage adapté pour traiter au moins en partie la coupe comprenant de l'éthane et/ou du propane avec de la vapeur d'eau, et produire un effluent de vapocraquage comprenant de l'éthylène et/ou du propylène.

D'autres caractéristiques et avantages de l'invention des aspects précités, apparaîtront à la lecture de la description ci-après et d'exemples non limitatifs de réalisations, en se référant à la figure annexée et décrite ci-après.

### Liste des figures

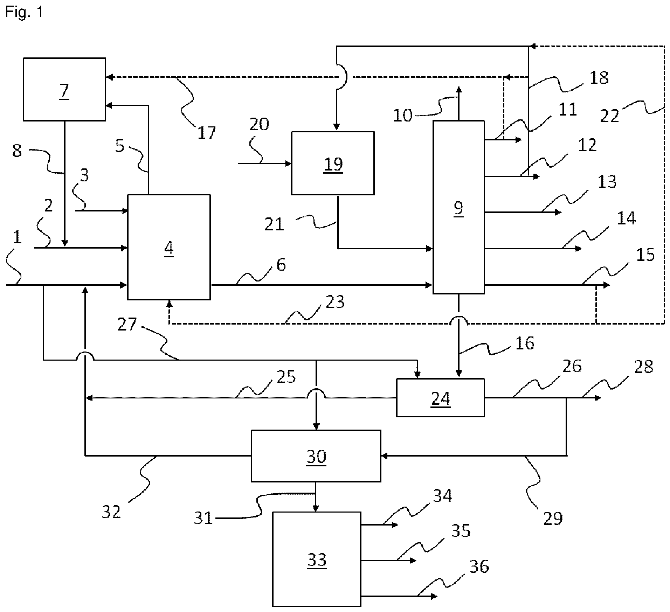

La figure 1 montre un schéma d'un procédé NCC selon la présente invention pour la production d'oléfines légères et optionnellement de BTX à partir de naphta.

### Description détaillée de l'invention

Des modes de réalisation selon les aspects précités vont maintenant être décrits en détail. Dans la description détaillée suivante, de nombreux détails spécifiques sont exposés afin de fournir une compréhension plus approfondie du dispositif. Cependant, il apparaîtra à l'homme du métier que le dispositif peut être mis en œuvre sans ces détails spécifiques. Dans d'autres cas, des caractéristiques bien connues n'ont pas été décrites en détail pour éviter de compliquer inutilement la description.

L'invention concerne un dispositif et un procédé NCC pour produire des oléfines légères et optionnellement des BTX à partir de charges de type Naphta. Un avantage de la présente invention est de fournir un dispositif et un procédé NCC permettant d'avoir des rendements importants en oléfines légères et en particulier propylène et éthylène par rapport aux dispositifs et procédés de craquage catalytique et permettant également de produire optionnellement des BTX.

Dans toute la suite du texte, on entend par oléfines légères, les oléfines ayant un nombre d'atomes de carbone compris entre 2 et 4. De préférence, les oléfines légères produites par le procédé selon l'invention sont l'éthylène et le propylène.

### Charge

Conformément à l'invention, la charge utilisée est une charge comprenant du naphta. Selon un ou plusieurs modes de réalisation, la charge consiste essentiellement en du naphta. Par exemple la charge peut comprendre au moins 90% poids, préférablement au moins 95% poids, très préférablement au moins 99% poids de naphta.

Selon un ou plusieurs modes de réalisation, le naphta est une charge essence comprenant et de préférence constituée de composés hydrocarbures ayant 4 à 15 atomes de carbone, de préférence 5 à 14 atomes de carbone.

Selon un ou plusieurs modes de réalisation, ladite charge essence présente un point d'ébullition initial compris entre 20 et 100°C et de préférence compris entre 20 et 80°C et de manière préférée entre 25 et 60°C et un point d'ébullition final compris entre 80 et 250°C et de préférence entre 100 et 220°C et de manière préférée entre 100 et 180°C.

Selon un ou plusieurs modes de réalisation, ladite charge essence est de type naphta léger de point d'ébullition final de 80°C à 100°C ou de type naphta lourd de point initial de 80°C à 100°C et de point d'ébullition final de 150°C à 220°C.

Selon un ou plusieurs modes de réalisation, la charge essence comprend une teneur en paraffines (normales + iso) comprise entre 20 et 90% poids et de préférence entre 30 et 85% poids, une teneur en oléfines comprise entre 0 et 60% poids et de préférence entre 0 et 30% poids, de manière préférée entre 0 et 15% poids, une teneur en naphtènes comprise entre 5 et 70% poids et de préférence entre 15 et 50% poids, une teneur en aromatiques comprise entre 0 et 50% poids et de préférence entre 0 et 30% poids, de manière préférée entre 0 et 15% poids, les pourcentages poids étant exprimés par rapport à la masse totale de ladite charge et la somme des différents composants étant égale à 100%.

Selon un ou plusieurs modes de réalisation, la charge essence utilisée dans le procédé selon l'invention est paraffinique, c'est-à-dire qu'elle comprend majoritairement des iso- et des n-paraffines. De manière préférée, ladite charge comprend des paraffines ayant 4 à 11 atomes de carbone et de préférence 5 à 9 atomes de carbone.

On entend par charge paraffinique, une charge essence comprenant une teneur en paraffines (normales + iso) d'au moins 50% poids, telle que comprise entre 50 et 90% poids, par rapport à la masse totale de ladite charge. Selon un ou plusieurs modes de réalisation, la charge comprend au moins 60% poids, préférablement au moins 70% poids, très préférablement au moins 80% poids de paraffines.

Selon un ou plusieurs modes de réalisation, la charge essence comprend moins de 20% poids d'oléfines (ayant de préférence au moins 5 et/ou au moins 9 atomes de carbone), de préférence moins de 17% poids d'oléfines et de manière préférée moins de 15% poids d'oléfines.

Selon un ou plusieurs modes de réalisation, ladite charge essence est issue de la distillation directe du pétrole, dans ce cas la charge est un naphta dit « straight run » selon la terminologie anglo-saxonne et/ou issue d'un ou plusieurs procédés de production d'essence (tel que par exemple le procédé de craquage catalytique en lit fluidisé ou « FCC », le procédé de cokéfaction ou « delayed coker » ou « flexicoker » selon la terminologie anglo-saxonne, le procédé d'hydrocraquage), et/ou d'une purge de procédé tel que les procédés d'isomérisation. On retrouve communément cette charge sous l'appellation « Naphta ». Ladite charge essence peut éventuellement subir une étape de prétraitement de type hydrotraitement préalablement à son utilisation dans le dispositif et le procédé selon l'invention, par exemple de façon à limiter ou éliminer les impuretés azotées, soufrées et les dérivés oxygénés.

Selon un ou plusieurs modes de réalisation, ladite charge essence peut être issue d'un procédé Fisher-Tropsch avant ou après des étapes d'hydrotraitement et/ou d'hydro-isomérisation.

### Etape de craquage catalytique du naphta

En référence à la figure 1, le procédé selon la présente invention comprend une étape dite NCC de production d'oléfines légères par craquage catalytique d'un naphta, dans lequel la charge 1 est mise en contact avec un catalyseur 2, et un diluant 3 dans un réacteur NCC 4.

Selon un ou plusieurs modes de réalisation, les conditions opératoires de l'étape NCC sont les suivantes : une température comprise entre 500 et 700°C, de préférence entre 550 et 700°C, de manière encore plus préférée entre 580 et 685°C ; une pression totale absolue comprise entre 0,1 et 0,5 MPa et de préférence entre 0,1 et 0,4 MPa et de manière préférée entre 0,1 et 0,3 MPa ; un temps de contact entre la charge 1 et le catalyseur 2 compris entre 200 millisecondes (ms) et 20 secondes, de préférence entre 400 millisecondes et 15 secondes et de manière préférée entre 600 millisecondes et 10 secondes; et une PPHcharge (pression partielle des hydrocarbures dans la charge) comprise entre 0,02 et 0,3 MPa, de préférence entre 0,03 et 0,2 MPa et de manière préférée entre 0,04 et 0,15 MPa.

Selon un ou plusieurs modes de réalisation, la charge 1 est mise en contact avec le catalyseur 2 dans un réacteur opérant en lit fixe, en lit mobile ou en lit fluidisé, de préférence dans un réacteur opérant en lit fluidisé, très préférablement en lit fluidisé turbulent. Dans la présente demande, le terme « lit fluidisé turbulent » signifie un lit fluidisé gaz-solide dans lequel la fraction volumique de solide (particules de catalyseur) est comprise entre 0,2 et 0,5, préférablement entre 0,25 et 0,4.

Selon un ou plusieurs modes de réalisation, le diluant 3 est choisi parmi un gaz inerte tel que l'azote et/ou la vapeur d'eau. Selon un ou plusieurs modes de réalisation, le diluant 3 est de la vapeur d'eau. Selon un ou plusieurs modes de réalisation, le diluant 3 est de l'azote. Selon un ou plusieurs modes de réalisation, la quantité de diluant 3 est choisie pour obtenir la PPHcharge mentionnée ci-dessus et le diluant 3 est introduit à raison d'une quantité représentant 0,1 à 40% poids, de préférence 1 à 35% poids et de manière préférée comprise entre 1 et 30% poids par rapport à la masse de la charge 1. Selon un ou plusieurs modes de réalisation, la charge 1 est introduite dans le réacteur NCC 4 en mélange avec de la vapeur d'eau. La présence de vapeur d'eau permet de baisser la pression partielle de la charge 1 thermodynamiquement défavorable pour les réactions de craquage et d'améliorer la fluidisation et les transferts thermiques. Par ailleurs, la présence d'eau permet également de limiter les réactions de transfert d'hydrogène qui conduisent à la formation d'alcanes légers tels que le méthane, l'éthane et le propane non désirés.

Dans le réacteur NCC 4, la charge 1 est convertie par craquage catalytique en présence du catalyseur 2 en oléfines légères et en particulier en éthylène et en propylène, et optionnellement en BTX (Benzène, Toluène et Xylène).

Selon un ou plusieurs modes de réalisation, à l'issue de l'étape NCC, un catalyseur coké 5 sort du réacteur NCC 4 et est avantageusement séparé de l'effluent de craquage 6 dit effluent NCC produit lors de l'étape NCC, strippé et régénéré dans un régénérateur 7 pour produire un catalyseur régénéré 8 recyclé vers le réacteur NCC 4. Le catalyseur étant généralement coké, il peut être régénéré par brulage du coke dans le régénérateur 7 au contact d'air de combustion alimentant ce dernier. Selon la production de coke dans le réacteur NCC 4, une charge combustible externe peut être avantageusement injectée dans le régénérateur pour boucler le bilan thermique du procédé.

L'effluent NCC 6 comprend une fraction gazeuse contenant des concentrations importantes en oléfines légères (C2-C4) et en particulier d'éthylène (C2) et de propylène (C3), et une fraction dite C5+ qui selon les conditions choisies peut contenir des quantités importantes de BTX.

Spécifiquement, l'effluent NCC 6 obtenu comprend un mélange d'hydrogène, méthane, éthane, éthylène, propane, propylène, une coupe C4 comprenant butanes et butènes, et une coupe C5+ pouvant contenir notamment des aromatiques, tels que des BTX. L'effluent NCC 6 est ensuite avantageusement refroidi et séparé pour obtenir les oléfines légères et de préférence l'éthylène et le propylène.

### Catalyseur de l'étape NCC

Le catalyseur 2 peut être tout type de catalyseur de craquage catalytique, de préférence contenant au moins une zéolithe, telle qu'une zéolithe choisie parmi les zéolithes NU-86, ZSM-5, ZSM-11, beta, Y, ferriérite, ZSM-22, ZSM-23, EU-1, seule ou en mélange, de préférence parmi les zéolithes NU-86, ZSM-5, ZSM-11, beta, Y et ferriérite, seule ou en mélange, et très préférentiellement parmi les zéolithes Y, NU-86 et ZSM-5, seule ou en mélange.

Selon un ou plusieurs modes de réalisation, le catalyseur 2 comprend au moins un liant choisi parmi l'alumine, la silice, la silice-alumine, la magnésie, l'oxyde de titane, la zircone, les argiles et l'oxyde de bore, seul ou en mélange et de préférence parmi la silice, la silice-alumine et les argiles, seul ou en mélange.

Selon un ou plusieurs modes de réalisation, le catalyseur 2 comprend, en pourcentage massique :
- de 20 à 80% poids d'au moins un liant ;
- de 20 à 80% poids d'au moins une zéolithe choisie parmi les zéolithes Nu-86, ZSM-5, ZSM-11, beta, Y, ferriérite, ZSM-22, ZSM-23 et EU-1, seule ou en mélange ; et
- de 0 à 12% poids d'au moins un élément dopant, les pourcentages étant exprimés par rapport à la masse totale dudit catalyseur 2 et la somme des teneurs en lesdits éléments étant égale à 100%.

Selon un ou plusieurs modes de réalisation, l'au moins un élément dopant est choisi parmi le phosphore, le magnésium, le sodium, le potassium, le calcium, le fer, le bore, le manganèse, le lanthane, le cérium, le titane, le tungstène, le molybdène, le cuivre, le zirconium et le gallium, seul ou en mélange.

### Etapes de séparation de l'effluent NCC

L'effluent NCC 6 est envoyé vers une première section de séparation 9, dans laquelle l'effluent NCC 6 est fractionné. Selon un ou plusieurs modes de réalisation, l'effluent NCC 6 est (rapidement) refroidi (« quenched » selon la terminologie anglo-saxonne), par exemple par une étape de refroidissement avant d'être fractionné (non montrée dans la figure). Selon un ou plusieurs modes de réalisation, la première section de séparation 9 permet de séparer les fractions suivantes : hydrogène 10 ; méthane 11 ; éthane et/ou propane 12; éthylène 13 ; propylène 14 ; coupe C4 15 comprenant notamment butanes et butènes ; coupe C5+ 16 comprenant des paraffines, oléfines et aromatiques.

Selon un ou plusieurs modes de réalisation, le méthane 11, et optionnellement l'éthane et/ou le propane ainsi séparés, sont au moins en partie utilisés comme gaz combustible 17 (« fuel gas » selon la terminologie anglo-saxonne), alimentant le régénérateur 7 pour boucler le bilan thermique du procédé, les réactions de craquage ayant lieu dans le réacteur NCC 4 pouvant être très endothermiques.

Selon un ou plusieurs modes de réalisation, l'éthane et/ou le propane, seul ou en mélange (ligne 18), sont au moins en partie envoyés vers un four de vapocraquage 19 (« steam cracking » selon la terminologie anglo-saxonne). Selon un ou plusieurs modes de réalisation, l'éthane ou le propane ou le mélange 18 est additionné de vapeur d'eau 20 selon un ratio massique [vapeur d'eau 20]/[coupe comprenant de l'éthane et/ou du propane 12] entre 0,2 et 0,8 et de préférence entre 0,3 et 0,5, et subit un craquage à une température comprise entre 750 et 900°C et de préférence entre 780 et 850°C.

Selon un ou plusieurs modes de réalisation, l'effluent de vapocraquage 21 est enrichie en éthylène et/ou propylène par rapport à la coupe éthane et/ou propane (12). Selon un ou plusieurs modes de réalisation, l'effluent de vapocraquage 21 comprend une teneur en éthylène comprise entre 30 et 70% poids et de préférence entre 35 et 55% poids, une teneur en propylène comprise entre 5 et 25% poids et de préférence entre 5 et 15% poids, les pourcentages poids étant exprimés par rapport à la masse totale dudit effluent et la somme des différents composants étant égale à 100%. L'effluent de vapocraquage 21 est envoyé à la première section de séparation 9 après avoir été optionnellement refroidi à une température comprise entre 500 et 700°C, et de préférence entre 550 et 650°C.

Selon un ou plusieurs modes de réalisation, au moins une partie de la coupe C4 15 est recyclée vers le four de vapocraquage 19 (ligne 22) et/ou le réacteur NCC 4 (ligne 23). Selon un ou plusieurs modes de réalisation, au moins une partie de la coupe C4 15 est séparée en une coupe C4 oléfinique et une coupe C4 paraffinique, et au moins une partie de la coupe C4 paraffinique est recyclée vers le four de vapocraquage 19 et/ou le réacteur NCC 4.

Selon un ou plusieurs modes de réalisation, au moins une partie de la coupe C5+ 16 est envoyée vers une deuxième section de séparation 24 pour produire une coupe C5 25 (majoritairement composée de paraffines et oléfines) et une coupe C6+ 26 enrichie en aromatiques par rapport à la coupe C5+ 16.

Selon un ou plusieurs modes de réalisation, lorsque la charge 1 comprend des aromatiques, par exemple lorsque la charge 1 comprend plus de 15% poids d'aromatiques, de préférence plus de 25% poids, de manière préférée plus de 35% poids, tout ou partie de la charge 1 est avantageusement envoyée (ligne 27) à la deuxième section de séparation 24 de l'étape de séparation de l'effluent NCC en mélange avec au moins une partie de la coupe C5+ 16 pour produire la coupe C5 25 directement recyclé au réacteur NCC 4 et la coupe C6+ 26 riche en aromatiques. Ces configurations sont particulièrement intéressantes pour des charges très aromatiques car elles permettent d'extraire les aromatiques de la charge essence avant de l'envoyer dans le réacteur NCC.

Selon un ou plusieurs modes de réalisation, la deuxième section de séparation 24 comprend ou consiste en une colonne de distillation. En tête de la colonne de distillation, la coupe enrichie en coupe C5 25 est soutirée. Selon un ou plusieurs modes de réalisation, la coupe C5 25 comprend entre 80 et 100% poids des composés à 5 atomes de carbone, de préférence entre 90 et 99% poids, et de manière très préférée entre 95 et 99% poids. En fond de colonne, la coupe C6+ 26 est soutirée. Avantageusement, la coupe C6+ 26 comprend la majorité des composés aromatiques de la coupe C5+ 16.

Selon un ou plusieurs modes de réalisation, la coupe C5 25 est au moins en partie recyclée vers le réacteur NCC 4 de manière à augmenter le rendement en oléfines légères. Selon un ou plusieurs modes de réalisation, la coupe C5 25 recyclée au réacteur NCC 4 contient entre 5 et 40% poids, de préférence entre 5 et 30% poids d'oléfines. Les oléfines étant plus réactives aux réactions de craquage que les paraffines de même nombre de carbone, le recyclage de la coupe C5 25 riche en oléfines C5 vers le réacteur NCC 4 est favorable à l'augmentation de la production d'oléfines légères.

Avantageusement, la coupe C6+ 26 peut être au moins en partie retirée du procédé (ligne 28).

Selon un ou plusieurs modes de réalisation, au moins une partie de la coupe C6+ 26 est envoyée (ligne 29) vers une unité d'extraction des aromatiques 30 pour produire une coupe enrichie en aromatiques 31, de préférence enrichie en BTX (benzène, toluène et xylènes) et une coupe pauvre en aromatiques 32, de préférence pauvre en BTX. L'unité d'extraction des aromatiques 30 peut être mise en œuvre selon différents procédés connus de l'homme du métier. Selon un ou plusieurs modes de réalisation, l'unité d'extraction des aromatiques 30 comprend une section d'hydrogénation des oléfines pour produire un effluent d'hydrogénation pauvre en oléfines et comprenant des paraffines et des aromatiques ; et une section d'extraction pour séparer l'effluent d'hydrogénation et produire la coupe enrichie en aromatiques 31 et la coupe pauvre en aromatiques 32. Ainsi, selon un ou plusieurs modes de réalisation, la coupe pauvre en aromatiques 32 est également appauvrie en oléfines.

Selon un ou plusieurs modes de réalisation, lorsque la charge 1 comprend des aromatiques, par exemple lorsque la charge 1 comprend plus de 15% poids d'aromatiques, de préférence plus de 25% poids, de manière préférée plus de 35% poids, tout ou partie de la charge 1 peut être avantageusement envoyée (ligne 27) directement à l'unité d'extraction des aromatiques 30 en mélange avec au moins une partie de la coupe C6+ 26 pour réaliser l'extraction des aromatiques, de préférence des BTX, de tout ou partie de la charge 1. Ces configurations sont particulièrement intéressantes pour des charges très aromatiques car elles permettent d'extraire les aromatiques de la charge essence avant de l'envoyer dans le réacteur NCC.

Selon un ou plusieurs modes de réalisation, la coupe pauvre en aromatiques 32 est au moins en partie recyclée vers le réacteur NCC 4 de manière à augmenter le rendement en oléfines.

Selon un ou plusieurs modes de réalisation, au moins une partie de la coupe enrichie en aromatiques 31 est envoyée vers une unité de séparation des aromatiques 33 pour produire au moins : une fraction riche en benzène 34, une fraction riche en toluène 35, au moins une fraction riche en xylènes 36 (coupe de paraxylène, d'orthoxylène et/ou de métaxylène, et préférablement une coupe de paraxylène), et éventuellement au moins une coupe d'aromatiques ayant au moins 9 atomes de carbone.

L'unité de séparation des aromatiques 33 peut être mise en œuvre selon différents procédés connus de l'homme du métier. Selon un ou plusieurs modes de réalisation, l'unité de séparation des aromatiques 33 comprend un dispositif de conversion de composés aromatiques dit « complexe aromatique ». Au sein du complexe aromatique, le benzène et les alkyles aromatiques (toluène, xylènes, éthylbenzène et composés aromatiques à 9 atomes de carbone ou plus) y sont extraits au moyen d'un train de fractionnement. De plus, un complexe aromatique dispose généralement d'au moins une unité catalytique telle que les suivantes :
- l'isomérisation des composés aromatiques à 8 atomes de carbone - cette unité permet de convertir au moins en partie l'orthoxylène, le métaxylène et l'éthylbenzène en paraxylène ;
- la transalkylation permettant de produire des xylènes à partir d'un mélange de toluène (et optionnellement de benzène) et de composés aromatiques à 9 atomes de carbone ou plus, tels que les triméthylbenzènes et les tétraméthylbenzènes ; et
- la dismutation du toluène, qui permet de produire du benzène et des xylènes.

Un complexe aromatique dispose également généralement d'une section de séparation des C8-aromatiques par adsorption en lit mobile simulé pour produire un extrait (paraxylène et désorbant, tel que le toluène) et un raffinat (orthoxylène, métaxylène et éthylbenzène), ou une section de cristallisation dans lequel le paraxylène est isolé sous forme de cristaux du reste des composés d'un mélange comprenant des composés aromatiques à 8 atomes de carbone.

Dans la présente demande, le terme « comprendre » est synonyme de (signifie la même chose que) « inclure » et « contenir », et est inclusif ou ouvert et n'exclut pas d'autres éléments non récités. Il est entendu que le terme « comprendre » inclut le terme exclusif et fermé « consister ».

### Exemples

Conditions des tests :
- réacteur à lit fluidisé turbulent ;
- température : 682,2°C ;
- pression : 1,05 bar a (0,105 MPa a) ;
- diluant : azote ;
- PPHcharge : 0,64 bar (0,064 MPa) ;
- temps de contact : 3774,08 ms ;
- catalyseur zéolithique type ZSM-5 ; et
- charge : naphta léger avec les caractéristiques décrites sur la table 1.

**Tableau 1**

| | | |
|---|---|---|
| Masse volumique à 15°C | g/cm³ | 0,6687 |
| Masse molaire | g/mol | 81,82 |
| Distillation simulée | | |
| Point initial ASTM 2887 | °C | 30,2 |
| Point final ASTM 2887 | °C | 111,2 |
| N paraffines | % m/m | 36,1 |
| Iso paraffines | % m/m | 48,5 |
| Naphtènes | % m/m | 12,1 |
| Aromatiques | % m/m | 2,8 |
| Oléfines | % m/m | 0 |
| Benzène | % m/m | 1,72 |
| Toluène | % m/m | 1,0244 |

### Exemples A :

La table 2 décrit la comparaison des rendements issus de :
1) la première section de séparation 9 après passage dans le réacteur NCC 4 sans recyclage (non conforme à la présente invention) ;
2) la première section de séparation 9 après passage dans le réacteur NCC 4 (non conforme à la présente invention), avec :
   - envoi de la coupe C5+ 16 directement dans l'unité d'extraction des aromatiques 30 pour extraction de la coupe enrichie en aromatiques 31 et recyclage de la coupe pauvre en aromatiques 32 ;
3) la première section de séparation 9 après passage dans le réacteur NCC 4 (non conforme à la présente invention), avec :
   - envoi de la coupe C5+ 16 dans la deuxième section de séparation 24,
   - envoi de la coupe C5 25 dans le réacteur NCC 4, et
   - envoi de la coupe C6+ 26 dans l'unité d'extraction des aromatiques 30 pour extraction de la coupe enrichie en aromatiques 31 et recyclage de la coupe pauvre en aromatiques 32 ; et
4) la première section de séparation 9 après passage dans le réacteur NCC 4 (conforme à la présente invention), avec :
   - envoi de la coupe C5+ 16 dans la deuxième section de séparation 24,
   - envoi de la coupe C5 25 dans le réacteur NCC 4,
   - envoi de la coupe C6+ 26 dans l'unité d'extraction des aromatiques 30 pour extraction de la coupe enrichie en aromatiques 31 et recyclage de la coupe pauvre en aromatiques 32,
   - envoi de l'éthane et du propane 12 dans le four de vapocraquage 19, et
   - envoi de l'effluent de vapocraquage 21 dans la première section de séparation 9.

**Tableau 2**

| # | 1 | 2 | 3 | 4 |
|---|---|---|---|---|
| Rendements | % poids | % poids | % poids | % poids |
| Ethylène | 22,2 | 28,0 | 28,6 | 39,3 |
| Propylène | 18,8 | 23,8 | 24,2 | 25,0 |
| Essence | 28,7 | 0,0 | 0,0 | 0,0 |
| BTX | 0,0 | 10,1 | 10,1 | 10,4 |
| Total oléfines légères (C2 and C3) | 41,0 | 51,8 | 52,8 | 64,3 |
| Total oléfines légères (C2 and C3) + BTX | 41,0 | 61,8 | 62,8 | 74,7 |

### Exemples B :

La table 3 décrit la comparaison des rendements issus de :
1) la première section de séparation 9 après passage dans le réacteur NCC 4 sans recyclage (non conforme à la présente invention) ;
5) la première section de séparation 9 après passage dans le réacteur NCC 4 (conforme à la présente invention), avec :
   - envoi de l'éthane et du propane 12 dans le four de vapocraquage 19, et
   - envoi de l'effluent de vapocraquage 21 dans la première section de séparation 9 ; et
4) la première unité de séparation 9 après passage dans le réacteur NCC 4 (conforme à la présente invention), avec :
   - envoi de la coupe C5+ 16 dans la deuxième section de séparation 24,
   - envoi de la coupe C5 25 dans le réacteur NCC 4,
   - envoi de la coupe C6+ 26 dans l'unité d'extraction des aromatiques 30 pour extraction de la coupe enrichie en aromatiques 31 et recyclage de la coupe pauvre en aromatiques 32,
   - envoi de l'éthane et du propane 12 dans le four de vapocraquage 19, et
   - envoi de l'effluent de vapocraquage 21 dans la première section de séparation 9.

**Tableau 3**

| # | 1 | 5 | 4 |
|---|---|---|---|
| Rendements | % poids | % poids | % poids |
| Ethylène | 22,2 | 31,1 | 39,3 |
| Propylène | 18,8 | 19,8 | 25,0 |
| Essence | 28,7 | 20,4 | 0,0 |
| BTX | 0,0 | 0,0 | 10,4 |
| Total oléfines légères (C2 and C3) | 41,0 | 50,9 | 64,3 |
| Total oléfines légères (C2 and C3) + BTX | 41,0 | 50,9 | 74,7 |

## Revendications

1. Procédé de production d'oléfines ayant un nombre d'atomes de carbone compris entre 2 et 4 par craquage catalytique, comprenant les étapes suivantes :
- mettre en contact toute ou partie d'une charge comprenant du naphta (1), un catalyseur (2) et un diluant (3) dans un réacteur de craquage catalytique de naphta (4) pour convertir au moins en partie la charge comprenant du naphta (1) en oléfines ayant un nombre d'atomes de carbone compris entre 2 et 4 et produire un effluent de craquage (6) ;
- envoyer l'effluent de craquage (6) dans une première section de séparation (9) pour produire au moins les fractions suivantes :
∘ coupe comprenant de l'éthane et/ou du propane (12),
∘ coupe comprenant de l'éthylène (13), et
∘ coupe comprenant du propylène (14) ; et
- envoyer au moins en partie la coupe comprenant de l'éthane et/ou du propane (12) dans un four de vapocraquage (19) alimenté par de la vapeur d'eau (20) pour produire un effluent de vapocraquage (21) comprenant de l'éthylène et/ou du propylène.

2. Procédé de production d'oléfines selon la revendication 1, dans lequel la coupe comprenant de l'éthane et/ou du propane (12) est additionnée à la vapeur d'eau (20) selon un ratio massique [vapeur d'eau (20)]/[coupe comprenant de l'éthane et/ou du propane (12)] entre 0,2 et 0,8, et subit un craquage à une température comprise entre 750 et 900°C dans le four de vapocraquage (19).

3. Procédé de production d'oléfines selon la revendication 1 ou la revendication 2, dans lequel l'effluent de vapocraquage (21) est envoyé dans la première section de séparation (9).

4. Procédé de production d'oléfines selon l'une quelconque des revendications précédentes, dans lequel la première section de séparation (9) produit au moins une coupe comprenant des composés comprenant 4 atomes de (15), et dans lequel au moins une partie de la coupe comprenant des composés comprenant 4 atomes de carbone (15) est recyclée vers le four de vapocraquage (19).

5. Procédé de production d'oléfines selon l'une quelconque des revendications précédentes, dans lequel la première section de séparation (9) produit au moins une coupe comprenant des composés comprenant 4 atomes de carbone (15), et dans lequel au moins une partie de la coupe comprenant des composés comprenant 4 atomes de carbone (15) est recyclée vers le réacteur de craquage catalytique de naphta (4).

6. Procédé de production d'oléfines selon l'une quelconque des revendications précédentes, dans lequel le réacteur de craquage catalytique de naphta (4) est opéré en lit fluidisé turbulent.

7. Procédé de production d'oléfines selon l'une quelconque des revendications précédentes, dans lequel le catalyseur (2) comprend au moins une zéolithe, et dans lequel le réacteur de craquage catalytique de naphta (4) est utilisé selon les conditions opératoires suivantes : une température comprise entre 500 et 700°C ; une pression totale absolue comprise entre 0,1 et 0,5 MPa ; un temps de contact entre la charge comprenant du naphta (1) et le catalyseur (2) compris entre 200 millisecondes et 20 secondes ; et une pression partielle des hydrocarbures dans la charge comprenant du naphta (1) comprise entre 0,02 et 0,3 MPa.

8. Procédé de production d'oléfines selon l'une quelconque des revendications précédentes, dans lequel un catalyseur coké (5) est extrait du réacteur de craquage catalytique de naphta (4), est séparé de l'effluent de craquage (6), est strippé et est régénéré dans un régénérateur (7) pour produire un catalyseur régénéré (8) recyclé dans le réacteur de craquage catalytique de naphta (4).

9. Procédé de production d'oléfines selon la revendication 8, dans lequel la première section de séparation (9) produit au moins une coupe comprenant du méthane (11), et dans lequel la coupe comprenant du méthane (11) est au moins en partie utilisée comme gaz combustible (17) alimentant le régénérateur (7).

10. Procédé de production d'oléfines selon la revendication 8 ou la revendication 9, dans lequel la première section de séparation (9) produit au moins une coupe comprenant de l'éthane et/ou du propane (12), et dans lequel la coupe comprenant de l'éthane et/ou du propane (12) est au moins en partie utilisée comme gaz combustible (17) alimentant le régénérateur (7).

11. Procédé de production d'oléfines selon l'une quelconque des revendications précédentes, dans lequel la première section de séparation (9) produit au moins une coupe comprenant des composés comprenant au moins 5 atomes de carbone (16) comprenant des paraffines, des oléfines et des aromatiques, le procédé comprenant en outre les étapes suivantes :
- envoyer au moins une partie de la coupe comprenant des composés comprenant au moins 5 atomes de carbone (16) dans une deuxième section de séparation (24) pour produire les fractions suivantes :
∘ coupe comprenant des composés comprenant 5 atomes de carbone (25), et
∘ coupe comprenant des composés comprenant au moins 6 atomes de carbone (26) riche en aromatiques ;
- envoyer au moins une partie de la coupe comprenant des composés comprenant au moins 6 atomes de carbone (26) dans une unité d'extraction des aromatiques (30) pour produire les fractions suivantes :
∘ coupe enrichie en aromatiques (31), et
∘ coupe pauvre en aromatiques (32) ; et
- recycler au moins en partie la coupe comprenant des composés comprenant 5 atomes de carbone (25) dans le réacteur de craquage catalytique de naphta (4).

12. Procédé de production d'oléfines selon la revendication 11, dans lequel la coupe pauvre en aromatiques (32) est recyclée au moins en partie dans le réacteur de craquage catalytique de naphta (4).

13. Procédé de production d'oléfines selon la revendication 11 ou la revendication 12, dans lequel la charge comprenant du naphta (1) comprend des aromatiques et dans lequel toute ou partie de la charge comprenant du naphta (1) est envoyée dans la deuxième section de séparation (24) ou dans l'unité d'extraction des aromatiques (30).

14. Procédé de production d'oléfines selon l'une quelconque des revendications 11 à 13, dans lequel l'unité d'extraction des aromatiques (30) comprend une section d'hydrogénation des oléfines pour produire un effluent d'hydrogénation pauvre en oléfines et comprenant des paraffines et des aromatiques ; et une section d'extraction pour séparer l'effluent d'hydrogénation et produire la coupe enrichie en aromatiques (31), et la coupe pauvre en aromatiques (32).

15. Dispositif de production d'oléfines ayant un nombre d'atomes de carbone compris entre 2 et 4 et d'aromatiques par craquage catalytique, comprenant les unités suivantes :
- un réacteur de craquage catalytique de naphta (4) adapté pour mettre en contact toute ou partie d'une charge comprenant du naphta (1), un catalyseur (2) et un diluant (3), convertir au moins en partie la charge comprenant du naphta (1) en oléfines ayant un nombre d'atomes de carbone compris entre 2 et 4 et produire un effluent de craquage (6) ;
- une première section de séparation (9) adaptée pour traiter l'effluent de craquage (6) et produire au moins les fractions suivantes :
∘ coupe comprenant de l'éthane et/ou du propane (12),
∘ coupe comprenant de l'éthylène (13), et
∘ coupe comprenant du propylène (14) ;
- un four de vapocraquage (19) adapté pour traiter au moins en partie la coupe comprenant de l'éthane et/ou du propane (12) avec de la vapeur d'eau (20), et produire un effluent de vapocraquage (21) comprenant de l'éthylène et/ou du propylène.
